Europäisches Patentamt

**European Patent Office**

Office européen des brevets

(11) Numéro de publication: **0 125 981**
**B1**

(12) **FASCICULE DE BREVET EUROPEEN**

(45) Date de publication du fascicule du brevet:
**11.03.87**

(51) Int. Cl.⁴: **C 12 N 9/10**

(21) Numéro de dépôt: **84400909.2**

(22) Date de dépôt: **03.05.84**

(54) **Procédé de purification de la dextrane-saccharase.**

(30) Priorité: **06.05.83 FR 8307650**

(43) Date de publication de la demande:
**21.11.84 Bulletin 84/47**

(45) Mention de la délivrance du brevet:
**11.03.87 Bulletin 87/11**

(84) Etats contractants désignés:
**BE DE GB IT NL SE**

(56) Documents cités:
**GB - A - 2 079 290**
**US - A - 4 144 130**

**BIOSIS ARTICLE BIOLOGICAL ABSTRACTS/RRM
21051377, Philadelphia, US; A.W. MILLER et al.:
"Stabilization of dextran sucrase from
leuconostoc-mesenteroides NRRL B-512-F by nonionic
detergents and poly ethylene glycol"
CARBOHYDRATE RESEARCH, vol. 101, no. 1, 1982,
pages 57-74, Elsevier Scientific Publishing Company,
Amsterdam, NL; G.L. COTE et al.: Isolation and partial
characterization of an extracellular clucansucrase from
Leuconostoc mesenteroides NRRLB-1355 that
synthesizes an alternating (1-6),(1-3)-alpha-D-glucan"
BIOTECHNOLOGY AND BIOENGINEERING, vol. 23, no.
9, 1981, pages 2027-2037, John Wiley & Sons, Inc, New
York, US; P. MONSAN et al.: "On the production of
dextran by free and immobilized dextransucrase"**

(73) Titulaire: **SOCIETE NATIONALE ELF AQUITAINE, Tour
Aquitaine, F-92400 Courbevoie (FR)**

(72) Inventeur: **Paul, François, 28 rue du Vivier,
F-31650 Saint-Orens (FR)**
Inventeur: **Monsan, Pierre, Renoufail-Mondonville,
F-31700 Blagnac (FR)**
Inventeur: **Auriol, Daniel, Rue Henri Barbusse,
F-66460 Maury (FR)**

(74) Mandataire: **Ohresser, François, Société Nationale Elf
Aquitaine Division Proprieté Industrielle Tour Elf,
F-92078 Paris La Défense Cédex 45 (FR)**

## Description

La présente invention a pour objet un procédé de purification et de concentration simultanées d'une enzyme, la dextrane-saccharase à partir d'un surnageant de culture de la bactérie productrice de l'enzyme.

Les dextranes sont des polymères du glucose qui sont obtenus par transformation enzymatique du saccharose. Ces polysaccharides ont diverses applications industrielles en fonction de leur masse molaire. On peut citer comme exemples l'utilisation des dextranes de haute masse molaire dans l'industrie pétrolière en tant qu'agent viscosigène, l'utilisation des dextranes de masse molaire moyenne dans l'industrie alimentaire et celle des dextranes de faible masse molaire dans l'industrie pharmaceutique.

De nombreuses souches bactériennes faisant partie des genres LACTOBACILLUS, STREPTOCOC-CUS ou LEUCONOSTOC possèdent une activité dextrane-saccharase. Cependant parmi celles-ci, la souche de LEUCONOSTOC MESENTEROIDES NRRL B 512 (F) qui excrète l'enzyme dextrane-saccharase, lorsqu'elle est cultivée en présence de saccharose, présente des propriétés particulièrement intéressantes de stabilité, une productivité importante et un caractère non pathogène, qui ont permis de la mettre en œuvre sur le plan industriel.

De plus, la dextrane-saccharase issue de cette souche bactérienne produit un dextrane linéaire très peu ramifié (95% de liaisons glycosidiques α 1 → 6) et soluble dans l'eau, qualités qui en font un produit industriel très intéressant.

Les procédés industriels actuels de synthèse enzymatique des dextranes mettant en œuvre la dextrane-saccharase de LEUCONOSTOC MESENTEROIDES B 512 (F) présentent toutefois des inconvénients.

En effet, le procédé industriel consiste à produire l'enzyme dextrane-saccharase dans des fermenteurs de très grande taille. L'enzyme une fois excrétée par la bactérie, L. MESENTEROIDES NRRL B 512 (F), peu ou pas purifiée, est mise en contact avec son substrat, la saccharose, il y a alors production de dextrane brut dont on ne peut contrôler efficacement les conditions de synthèse.

Ce dextrane est ensuite soumis à divers traitements physico-chimiques pour sa récupération et purification, le rendement global de la réaction est ainsi relativement faible.

La présente invention a pour but un procédé économique de purification de l'enzyme dextrane-saccharase de LEUCONOSTOC-MESENTEROIDES, pouvant en outre, s'intégrer aisément dans un procédé de production de l'enzyme fonctionnant en continu. La productivité élevée ainsi obtenue alliée à une technique de purification, faisant l'objet de la présente invention, simple et remarquablement efficace justifie l'utilisation de cette préparation enzymatique au plan industriel et permet en outre par un contrôle précis des conditions de synthèse l'obtention de divers types de dextranes suivant le domaine d'application envisagé.

Les développements récents de procédés de synthèse enzymatique ont amené les chercheurs à se pencher sur les problèmes de purification des enzymes.

De nombreuses techniques de purification sont utilisées dans ce but, on peut citer à titre d'exemples la filtration et ses variantes micro- et ultra-filtration, la centrifugation, la chromatographie, la précipitation, l'extraction liquide-liquide, qui a eu récemment des développements mettant en œuvre le phénomène dit de démixtion de phase. A ce sujet on peut citer les travaux de M.R. KULA (Applied Biochemistry and Bioengineering Vol. 2 pp 71.95 Academic Press 1977 et brevet US 4 144 130) qui décrivent une technique de purification de diverses enzymes mettant en œuvre la démixtion de phase entre deux polymères, le polyethylène glycol et le dextrane qui, à certaines concentrations en solution aqueuse, deviennent incompatibles provoquant ainsi l'apparition de deux phases liquides dinsctites entre lesquelles se partagent les substances présentes dans le milieu en fonction de leur solubilité ou de leur affinité pour l'une ou l'autre des 2 phases dans les conditions opératoires. Le milieu de culture de la souche productrice de la dextrane saccharase contient d'autres enzymes extra cellulaires qu'il est important de pouvoir éliminer; en particulier deux enzymes saccharolytiques capables d'utiliser le saccharose sont excrétées par les souches de type LEUCONOSTOC MESENTEROIDES, la levane-saccharase et l'invertase. Ces deux enzymes sont particulièrement gênantes: d'une part elles diminuent le rendement en dextrane, d'autre part elles empêchent le contrôle satisfaisant de la production de dextranes. Ces deux activités contaminantes peuvent être schématisées comme suit:

$$\text{Saccharose} \xrightarrow[\text{levane-saccharase}]{} \text{levane + glucose}$$

$$\text{Saccharose} \xrightarrow[\text{invertase}]{} \text{glucose + fructose}$$

La présence de glucose révèle une activité contaminante: en effet, une solution pure de dextrane-saccharase en présence de saccharose ne produit pas de glucose sous forme libre. L'analyse du pourcentage relatif de glucose et de fructose apparaissant en cours de purification lors de la mesure de l'activité dextrane-saccharase est donc un critère de pureté de l'enzyme.

La présente invention a pour objet de pallier les inconvénients précédents en proposant un procédé efficace, simple et économique de purification et de concentration simultanées de l'enzyme dextrane-saccharase mettant en œuvre de façon originale l'extraction liquide-liquide par démixtion de phase.

La présente invention a pour objet un procédé de purification et de concentration simultanées de l'enzyme dextrane-saccharase à partir du milieu de culture sur saccharose de la bactérie LEUCONOSTOC MESENTEROIDES productrice de l'enzyme extra-cellulaire, ledit milieu contenant du dextrane, caractérisé en ce que dans une première étape on ajoute au milieu une solution aqueuse d'un polyéther à une quantité telle qu'au sein du milieu apparaissent deux phases non miscibles et on maintient ce milieu sous agitation afin d'assurer un contact intime et prolongé entre les deux polymères et les substances biochimiques, puis dans une deuxième étape on sépare la phase inférieure du milieu, riche en dextrane, de la phase supérieure, riche en polyether, la phase inférieure constituant une préparation enzymatique enrichie en dextrane-saccharase.

Le procédé se traduit par une séparation en deux phases du milieu de culture:
— une phase lourde riche en dextrane qui contient l'enzyme dextrane-saccharase concentrée et purifiée avec un coefficient de partage

$$K = \frac{C \text{ phase supérieure}}{C \text{ phase inférieure}}$$

voisin de zéro;
— une phase plus légère riche en polyéther qui contient les impuretés, notamment les protéines et les sucres ainsi que les activités enzymatiques contaminantes, phase qui est éliminée.

A titre d'exemple de polyéthers utilisables dans le procédé de l'invention on peut citer:
— le polyéthylène glycol (P.E.G.)
— le polypropylèneglycol
et leurs dérivés tels que:
— le méthoxypolyéthylèneglycol
— le triméthylamine polyéthylèneglycol
— le sulfonate de polyéthylèneglycol etc.

La masse molaire moyenne de polyéther utilisé sera généralement comprise entre 400 et 20 000. Cette masse molaire sera choisie en fonction de la nature du dextrane présent dans le milieu.

La quantité de polyéther ajoutée sera généralement telle que la teneur du milieu en polyalcool après addition de celui-ci sera comprise entre 1 et 15% en poids. La teneur optimale sera déterminée en fonction de la teneur en dextrane dans le milieu.

Le procédé est mis en œuvre sensiblement à la température ambiante c'est-à-dire à une température au plus égale à 30°C. En outre, le pH du milieu sera généralement compris entre 4, 5 et 7.

Il sera avantageux pour faciliter la mise en œuvre du procédé d'éliminer au préalable du milieu les cellules par exemple par centrifugation.

A la suite des travaux de M.R. KULA mentionnés ci-dessus il a été montré qu'il était possible de purifier les enzymes en utilisant le phénomène de démixtion de phase. Cependant, il apparaît clairement que lors du processus de purification les enzymes se répartissent entre les deux phases mais que la fraction purifiée d'enzyme s'obtient uniquement à partir de la phase supérieure PEG, la phase inférieure dextrane servant essentiellement à capter les débris cellulaires et impuretés présents dans le milieu.

Or, il a été constaté par la demanderesse que dans le procédé de l'invention la quasi-totalité de l'enzyme dextrane-saccharase est entraînée dans la phase inférieure dextrane alors que les autres activités enzymatiques contaminantes passent préférentiellement dans la phase supérieure polyéther. La phase inférieure dextrane peut ainsi servir de source de préparations enzymatiques dextrane-saccharase et cela d'autant plus que cette purification s'accompagne d'une concentration très importante, le volume de la phase inférieure correspondant à quelques % du volume du milieu soumis à la démixtion de phase.

Naturellement, le procédé pourra être mis en œuvre en deux ou plusieurs étapes au cours desquelles la phase inférieure issue de l'étape antérieure sera soumise à une nouvelle extraction par démixtion de phase dans des conditions opératoires analogues, après l'avoir, le cas échéant, pour des raisons de facilité de mise en œuvre, légèrement diluée.

Ce procédé de purification en plusieurs étapes peut être réalisé en continu dans une colonne d'extraction liquide-liquide fonctionnant à contre-courant.

Le procédé de l'invention est illustré par les exemples ci-après, pour l'interprétation desquels les définitions ci-dessous sont nécessaires.

*Definition des activités enzymatiques*

A partir du saccharose, trois réactions enzymatiques principales peuvent se produire:

$$— n\ G\text{-}F \xrightarrow[\text{dextrane-saccharase}]{} \text{Dextrane} + nF$$

$$— n\ G\text{-}F \xrightarrow[\text{levane-saccharase}]{} \text{Levane} + nG$$

$$— n\ G\text{-}F \xrightarrow[\text{invertase}]{} nG + nF$$

G-F: saccharose
G  : glucose
F  : fructose

La dextrane-saccharase libère exclusivement du fructose. La levane-saccharase libère exclusivement du glucose. L'invertase libère une quantité égale de fructose et glucose.

Si l'on observe une production de glucose au cours de la mesure de l'activité saccharolytique, elle résulte d'une activité contaminante, invertase ou levane-saccharase. Mais, dans le but de permettre une expression claire des résultats la production de glucose a été attribuée à une activité levane-saccharase qui est largement prépondérante. Ainsi l'activité levane-saccharase et l'activité dextrane-saccharase qui apparaissent dans les tableaux sont déterminées ainsi:

activité levane-saccharase (ULS): glucose total libéré

activité dextrane-saccharase (UDS): fructose total libéré

activité saccharolytique totale: fructose+glucose.

*Méthodes de mesure*

1. Mesure de l'activité enzymatique

Pour doser spécifiquement le glucose et le fructose, une méthode de dosage enzymatique (hexokinase/ATP, glucose-6-phosphate déshydrogénase/ $NADP^+$, phospho-gluco-isomérase) a été utilisée. L'activité saccharolytique totale est mesurée par dosage des sucres réducteurs totaux (glucose + fructose) au moyen du réactif au DNS (acide dinitro- salicylique); SUMNER, J.B. J. Biol Chem 47,5 1921).

### 2. Mesure des protéines

La méthode de Lowry a été utilisée LOWRY, O.H. ROSEBROUGH NM, FARR A.L., and R.M. RANDALL J. Biol. Chem. 193 (1951) 265-275.

### 3. Mesure des polymères (dextrane ou levane)

Le polyéthylèneglycol et les oligosacharides présents sont d'abord éliminés par ultrafiltration dans une cartouche de fibres creuses (Amicon) dont le seuil de coupure est de 100000, le polymère est ensuite dosé à l'aide du réactif à l'anthrone; SCOTT, T.A. and MELVIN, E.H. (1953) Anal. Chem. 25, 1656.

Le levane est dosé spécifiquement suivant la technique décrite par P. PERLOT. Thèse de Docteur-Ingénieur, Institut National des Sciences Appliquées, TOULOUSE (1980). Il a été vérifié que le dextrane n'interferait pas avec cette technique.

*Principe du calcul de l'activité:*

1 mg de saccharose (masse molaire 342) libère 0,474 mg sous forme de polymère (levane ou dextrane: masse molaire du motif monomérique: 162) et 0,256 mg de sucre libre (fructose ou glucose: masse molaire 180).

L'activité est mesurée en unités par ml de solution ou par mg de protéine. Une unité représente la conversion de 1 mg de saccharose par heure à 30°C dans le tampon acétate de sodium 20 mM à pH 5,2.

*Production du surnageant de culture*

Dans un fermenteur on fait croître la bactérie LEUCONOSTOC MESENTEROIDES NRRL B 512 (F) en présence de saccharose. Lors de la fermentation les enzymes saccharolytiques et notamment la dextrane-saccharase sont excrétées dans le milieu par la bactérie. L'enzyme dextrane-saccharase synthétise le dextrane dans le milieu.

La fermentation est alors arrêtée, les cellules présentes sont éliminées par centrifugation et le surnageant de culture est recueilli pour être soumis au procédé de purification.

*Exemple 1*

Dans cet exemple on a traité un surnageant de culture obtenu comme indiqué ci-dessus de 500 ml de volume en y ajoutant progressivement 175 ml d'une solution aqueuse de PEG 1500 à 50% (poids/volume), l'homogénéisation du milieu étant assurée par une agitation magnétique.

Ensuite on sépare la phase inférieure dextrane de la phase supérieure PEG. Des fractions de ces phases sont prélevées pour analyse. On reprend ensuite 2,7 ml de la phase dextrane que l'on dilue à 45 ml par une solution tampon acétate de sodium 20 mM pH 5,2 puis on pratique une deuxième séparation par démixtion de phase en y ajoutant 11,7 ml de la solution de PEG.

Les résultats obtenus sont réunis dans le tableaux ci-après.

Ces résultats (1A et 1B) ont été obtenus après la première étape du procédé. Ils mettent en évidence la présence de levanes en quantité non négligeable (35%) dans le surnageant de fermentation ce qui indique la présence de levane-saccharase dans le surnageant. En outre ce levane passe préférentiellement dans la phase PEG (90%) au contraire du dextrane. Les résultats obtenus et résumés dans le tableau 1B montrent que les sucres simples glucose et fructose passent préférentiellement dans la phase PEG

$$\text{Les coefficients de } K = \frac{\text{(C phase supérieure PEG)}}{\text{(C phase inférieure dextrane)}}$$
partage

sont respectivement de 0,84 pour le glucose et de 0,65 pour le fructose (voir tableau 1C ci-après).

On peut constater à l'examen des résultats du tableau 1C un effet de concentration puisque la presque totalité de l'activité dextrane-saccharase (90%) est récupérée dans un volume correspondant à environ 5% du volume de la solution de départ.

En outre, on observe que la démixtion réalisée à partir du surnageant de culture se caractérise par la récupération dans la phase inférieure de seulement 53% du dextrane présent dans la solution de départ qui cependant contient 90% de l'activité dextrane-saccharase.

Les résultats résumés dans le tableau 1D montrent qu'au cours de la première démixtion l'essentiel de l'activité dextrane-saccharase passe dans la phase inférieure dextrane alors que la levanesaccharase se répartit également entre les deux phases, les coefficients de partage étant respectivement 0,00253 pour la dextrane-saccharase et 0,033 pour le levane-saccharase. Au cours de la deuxième démixtion toute l'activité dextrane-saccharase passe dans la phase dextrane (coefficient de partage ∼ 0).

*Exemple 2*

Cet exemple est destiné à illustrer la purification de la dextrane-saccharase par démixtion de phase en deux étapes et cela en présence d'une protéine ajoutée (albumine bovine) afin d'étudier son influence sur le procédé de purification. Au cours de la première étape on ajoute à 200 ml d'un surnageant de culture 4144 mg d'albumine bovine. On traite alors le surnageant selon le processus en deux étapes décrit à l'exemple 1. Lors de la première étape on ajoute 52 ml d'une solution de PEG 1500 à 50% poids/volume. Au cours de la deuxième étape, on reprend 6 ml de la phase dextrane issue de la première étape et les dilue à 33 ml au moyen de la solution tampon utilisée dans l'exemple 1, et on pratique la démixtion de phase par addition de 10,3 ml de PEG. Les résultats obtenus sont résumés dans le tableau 2. Ils montrent une concentration dans la phase dextrane de l'activité spécifique dextrane-saccharase en dépit de la présence des protéines qui sont éliminées de la phase dextrane dans de fortes proportions (environ 90%).

*Exemple 3*

Dans cet exemple on traite selon le procédé décrit dans l'exemple 1, mais ne comportant qu'une étape, 279 ml d'un surnageant de culture, auquel on a ajouté 170000 unités invertase exogène, par 89 ml de PEG. Cette addition d'unités invertase a pour but d'étudier l'influence de la présence d'invertase excédentaire sur la qualité de la purification. Les résultats sont résumés dans le tableau 3A. On peut constater que la présence en excès des unités invertase ne modifie en rien la purification de la dextrane-saccharase dans la phase dextrane, plus de 97% des unités invertase passant dans la phase supérieure PEG.

Les rendements sont indiqués dans le tableau 3B les coefficients de partage obtenus sont respectivement de 0,002 pour le dextrane-saccharase et de 1,37 pour l'invertase.

*Exemple 4*

Dans cet exemple on a traité par le procédé de purification en une étape 40 ml d'une solution, déjà purifiée, de dextrane-saccharase, en présence d'invertase exogène en ajoutant au milieu 13,5 ml de la solution PEG.

Les résultats obtenus sont résumés dans le tableau 4. On constate, comme dans l'exemple 3 précédent, qu'il ne reste plus d'activité dextrane-saccharase dans la phase PEG, alors que l'essentiel de l'activité invertase subsiste dans la phase supérieure PEG.

*Exemple 5*

Dans cet exemple on a traité selon le procédé de l'invention 150 ml d'un surnageant de fermentation, mais en lui appliquant cinq démixtions successives ce qui sur le plan quantitatif est représentatif de ce qu'on peut obtenir dans une colonne d'extraction fonctionnant en continu. Les résultats obtenus sont résumés dans le tableau 5A. On peut constater que pratiquement toute l'activité dextrane-saccharase est restée dans la phase dextrane avec un coefficient de concentration de plus de 20.

En outre, plus de 75% des unités levane-saccharase ont été éliminées. L'activité spécifique a été multipliée par plus de mille. Le coefficient de partage de la dextrane-saccharase est voisin de 0,002.

Les rendements sont illustrés dans le tableau 5B. Il faut enfin observer que la concentration et la purification s'effectuent dans un environnement stabilisant qui s'accompagnent d'une élimination rapide du saccharose résiduel issue de la fermentation et donc permet l'arrêt de la synthèse des dextranes faute de substrat; il est en effet important de limiter au maximum la concentration de dextrane dans la solution enzymatique.

### TABLEAU 1A

|  | Vol (ml) | Polysaccharides totaux (g) | Levane (g) | Levane % (poids) |
|---|---|---|---|---|
| Surnageant de fermentation | 500 | 4,65 | 1,64 | 35 |
| Phase PEG | 655 | 1,9 | 1,47 | 75 |
| Phase dextrane | 20 | 2,45 | | |

### TABLEAU 1B

|  | Volume (ml) | Glucose (%) | Fructose (%) |
|---|---|---|---|
| Surnageant de fermentation | 500 | 100 | 100 |
| Phase PEG | 655 | 96,5 | 95,5 |
| Phase dextrane | 20 | 3,5 | 4,5 |

### TABLEAU 1C

| 1ère démixtion | Volume ml | Unités Saccharolytiques | U.D.S. (mg Saccharose par heure) | U.L.S. (mg Saccharose par heure) | Protéines mg | Activité spécifique U.D.S./mg |
|---|---|---|---|---|---|---|
| Solution de départ | 500 | 76 500 | 67 420 | 9 090 | 2 809 | 24 |
| Phase PEG (supérieure) | 655 | 9 381 | 5 045 | 4 336 | 2 402 | 2 |
| Phase dextrane (inférieure) | 20 | 64 688 | 60 710 | 3 978 | 110 | 555 |

TABLEAU 1C (suite)

| 2ème démixtion | Volume ml | Unités Saccharoly-tiques | U.D.S. (mg Saccharo-se par heure) | U.L.S. (mg Saccharo-se par heure) | Protéines mg | Activité spécifique U.D.S./mg |
|---|---|---|---|---|---|---|
| Solution de départ | 45 | 8 844 | 8 188 | 656 | 16,2 | 510 |
| Phase PEG (supérieure) | 54,3 | 97 | 1 | 96 | | |
| Phase dextrane (inférieure) | 2,4 | 7 294 | 6 816 | 478 | 2,7 | 2 559 |

TABLEAU 1D

| première démixtion | Rendement dex-trane-saccharase | | deuxième démixtion | Rendement dex-trane-saccharase |
|---|---|---|---|---|
| Surnageant de fermentation (solution à purifier) | 100 | | Solution de départ | 100 |
| Phase PEG (supérieure) | 7,5 | | Phase PEG (supérieure | — |
| Phase dextrane (inférieure) | 90 | | Phase dextrane (inférieure) | 83,5 |

TABLEAU 2

| 1ère démixtion | Volume ml | U.D.S. | U.L.S. | Protéines de départ mg | Albumine Exogène mg | Protéines Totales mg | Activité spécifique UDS/mg |
|---|---|---|---|---|---|---|---|
| Solution de départ | 200 | 26 968 | 3 632 | 1 106 | 4 144 | 5 250 | 5,2 |
| Phase PEG (supérieure) | 244 | ~0 | 952 | | | 4 628 | 0 |
| Phase dextrane (inférieure) | 18 | 25 513 | 2 732 | | | 292 | 87,4 |
| 2ème démixtion | | | | | | | |
| Solution de départ | 33 | 8 429 | 901 | 96 | 785 | 881 | 9,6 |
| Phase PEG (supérieure) | 40,7 | 37 | 18 | | | 674 | 0,1 |
| Phase dextrane (inférieure) | 2,6 | 7 268 | 911 | | | 222 | 32,7 |

TABLEAU 3A

| | Volume ml | U.D.S. | Unités Invertase | Protéines mg | Activité spéci-fique UDS/mg protéïne |
|---|---|---|---|---|---|
| Solution de départ | 279 | 30 413 | 169 354 | 2 020 | 15,1 |
| Phase PEG (supérieure) | 354,8 | 0 | 162 000 | 1 983 | ~ 0 |
| Phase dextrane (inférieure) | 13,2 | 28 927 | 4 378 | 173 | 164,3 |

TABLEAU 3B

| Rendement | Dextrane Saccharase | Invertase |
|---|---|---|
| Solution départ | 100 | 100 |
| Phase PEG (supérieure) | — | 96 |
| Phase dextrane (inférieure) | 95 | 2,6 |

TABLEAU 4

| | Volume ml | U.D.S. | Unités Invertase | Protéines mg | Activité spécifique U.D.S./mg |
|---|---|---|---|---|---|
| Solution de départ | 40 | 2 203 | 5 330 | 1,6 | 1 377 |
| Phase PEG (supérieure) | 51 | ~ 0 | 4 873 | — | — |
| Phase dextrane (inférieure) | 2,5 | 1 735 | 286 | 0,92 | 1 886 |

TABLEAU 5A

| | Volume ml | U.D.S. Unités dextrane-saccharase | U.L.S. Unités saccharase | Protéines mg | Activité spécifique U.D.S./mg |
|---|---|---|---|---|---|
| Surnageant de formation (solution à purifier) | 150 | 16 815 | 2 535 | 962 | 18 |
| Phase dextrane (inférieure) après 5 démixtions | 7 | 15 885 | 615 | 0,75 | 21 180 |

TABLEAU 5B

| | U.D.S. | Rendement U.D.S. | U.L.S. | Rendement U.L.S. |
|---|---|---|---|---|
| Solution de départ | 16 815 | 100 | 2 535 | 100 |
| Phase PEG | 721 | 4,3 | 1 815 | 71,6 |
| Phase dextrane après 5 démixtions | 15 885 | 94,5 | 615 | 24,3 |

**Revendications**

1. Procédé de purification et de concentration simultanées de l'enzyme dextrane-saccharase à partir d'un milieu de culture sur saccharose de la bactérie LEUCONOSTOC MESENTEROIDES productrice de l'enzyme extra-cellulaire, ledit milieu contenant du dextrane, caractérisé en ce que dans une première étape on ajoute au milieu une solution aqueuse d'un polyéther à une quantité telle qu'au sein du milieu apparaissent deux phases non miscibles et on maintient ce milieu sous agitation afin d'assurer un contact intime et prolongé entre les deux polymères et les substances bio-chimiques, puis, dans une deuxième étape, on sépare la phase inférieure du milieu, riche en dextrane, de la phase supérieure riche en polyéther, la phase inférieure constituant une préparation enzymatique enrichie en dextrane-saccharase.

2. Procédé selon l'invention 1, caractérisé en ce que le polyéther est un polyglycol de masse moléculaire comprise entre 4000 et 20000.

3. Procédé selon l'une des revendications 1 et 2, caractérisé en ce que la teneur du milieu en polyéther après addition est comprise entre 1 et 15% en poids.

4. Procédé selon l'une des revendications 1 à 3, caractérisé en ce qu'il est réalisé à température inférieure ou égale à 30°C et à un pH compris entre 4,5 et 7.

5. Procédé selon l'une des revendications 1 à 4, caractérisé en ce que le milieu de culture est préalablement traité pour l'élimination des cellules.

6. Procédé selon l'une des revendications 1 à 5, caractérisé en ce qu'il est réalisé en plusieurs étapes successives, la solution traitée à chaque étape étant constituée par la phase dextrane recueillie à l'issue de l'étape précédente.

7. Procédé selon la revendication 6, caractérisé en ce qu'il est réalisé en continu dans une colonne à extraction liquide-liquide fonctionnant à contre-courant.

8. Procédé selon l'une des revendications 1 à 7, caractérisé en ce qu'il est appliqué à la purification de l'enzyme dextrane-saccharase de la bactérie LEUCONOSTOC MESENTEROIDES NRRL B 512 (F).

## Patentansprüche

1. Verfahren zur gleichzeitigen Reinigung und Konzentrierung des Enzyms Dextransaccharase aus einem Saccharose-Kulturmedium des das extrazelluläre Enzym produzierenden Bakteriums Leuconostoc mesenteroides, wobei das Medium Dextran enthält, gekennzeichnet durch Versetzen des Mediums in einer ersten Stufe mit einer wässrigen Polyetherlösung in einer solchen Menge, dass im Medium zwei nicht mischbare Phasen auftreten, und Bewegen des Mediums, um einen eingehenden und andauernden Kontakt zwischen den beiden Polymeren und den biochemischen Substanzen zu gewährleisten, und in einer zweiten Stufe Abtrennen der unteren, dextranreichen Phase des Mediums von der oberen, polyetherreichen Phase, wobei die untere Phase ein mit Dextransaccharase angereichertes Enzympräparat ist.

2. Verfahren nach Anspruch 1, gekennzeichnet durch Verwendung eines Polyglykols mit einer Molekülmasse von 400 bis 20000 als Polyether.

3. Verfahren nach Anspruch 1 oder 2, gekennzeichnet durch die Verwendung eines Kulturmediums mit einem Polyethergehalt nach Zugabe von 1 bis 15 Gew.-%.

4. Verfahren nach einem der Ansprüche 1 bis 3, gekennzeichnet durch Anwendung einer Temperatur von 30°C oder darunter und eines pH-Wertes von 4,5 bis 7.

5. Verfahren nach einem der Ansprüche 1 bis 4, gekennzeichnet durch vorhergehende Behandlung des Kulturmediums zur Entfernung der Zellen.

6. Verfahren nach einem der Ansprüche 1 bis 5, dadurch gekennzeichnet, dass es in mehreren, aufeinander folgenden Stufen durchgeführt wird, wobei die in jeder Stufe behandelte Lösung die am Ende der vorhergehenden Stufe gewonnene Dextranphase ist.

7. Verfahren nach Anspruch 6, dadurch gekennzeichnet, dass es kontinuierlich in einer Flüssig-Flüssig-Extraktionssäule im Gegenstrom durchgeführt wird.

8. Verfahren nach einem der Ansprüche 1 bis 7, dadurch gekennzeichnet, dass es auf die Reinigung des Enzyms Dextransaccharase des Bakteriums Leuconostoc mesenteroides NRRL B 512 (F) angewendet wird.

## Claims

1. Method for the simultaneous purification and concentration of the enzyme dextrane-saccharase from a culture medium on saccharose of the bacterium LEUCONOSTOC MESENTEROIDES which produces the extra-cellular enzyme, the said medium containing dextrane, characterised in that in a first stage, an aqueous solution of a polyether is added to the medium in a quantity such that there appear within the medium two immiscible phases, the medium being maintained under agitation to ensure intimate and prolonged contact between the two polymers and the biochemical substances and then, in a second stage, the lower phase of the medium rich in dextrane is separated from the upper phase which is rich in polyether, the lower phase constituting an enzymatic preparation which is enriched in dextrane-saccharase.

2. Method according to claim 1, characterised in that the polyether is a polyglycol with a molecular mass comprised between 400 and 20,000.

3. Method according to one of claims 1 and 2, characterised in that the polyether medium content after addition is comprised between 1 and 15% by weight.

4. Method according to one of claims 1 to 3, characterised in that it is carried out at a temperature less than or equal to 30 degrees C and at a pH between 4.5 and 7.

5. Method according to one of claims 1 to 4, characterised in that the culture medium is previously treated for the elimination of cells.

6. Method according to one of claims 1 to 5, characterised in that it is carried out in a plurality of successive stages, the solution treated at each stage being constituted by the dextrane phase collected at emergence from the preceding stage.

7. Method according to claim 6, characterised in that it is carried out continuously in a liquid-liquid extraction column operating on a counter-current principle.

8. Method according to one of claims 1 to 7, characterised in that it is applied to purification of the dextrane-saccharose enzyme of the bacterium LEUCONOSTOC MESENTEROIDES NRRL B 512 (F).